# EUROPEAN PATENT SPECIFICATION

(11) **EP 4 168 805 B1**
(45) Date of publication and mention of the grant of the patent: **18.02.2026**
(21) Application number: 21736001.5
(22) Date of filing: 18.06.2021
(51) Int. Cl.: G01N 33/68, G01N 33/92

(54) **METHOD FOR DETERMINING WHETHER A SUBJECT HAS A DISEASE OR CONDITION OR IS AT RISK OF DEVELOPING A DISEASE OR CONDITION**
VERFAHREN ZUR BESTIMMUNG, OB EINE PERSON EINE KRANKHEIT ODER EINEN ZUSTAND HAT ODER EIN RISIKO ZUR ENTWICKLUNG EINER KRANKHEIT ODER EINES LEIDENS IST
PROCÉDÉ POUR DÉTERMINER SI UN SUJET PRÉSENTE UNE MALADIE OU UN PROBLÈME DE SANTÉ OU EST SUSCEPTIBLE DE DÉVELOPPER UNE MALADIE OU UN PROBLÈME DE SANTÉ

(30) Priority: 18.06.2020 FI 20205648
(43) Date of publication of application: 26.04.2023
(73) Proprietor: NIGHTINGALE HEALTH OYJ, 00300 Helsinki (FI)
(72) Inventor: WÜRTZ, Peter, 00300 Helsinki (FI); SOININEN, Pasi, 00300 Helsinki (FI); KANGAS, Antti, J, 00300 Helsinki (FI); NYBO, Kristian, 00300 Helsinki (FI); JULKUNEN, Heli, 00300 Helsinki (FI); CICHONSKA, Anna, 00300 Helsinki (FI)
(74) Representative: Papula Oy
(86) International application number: PCT/FI2021/050467
(87) International publication number: WO 2021/255346

(56) References cited:
- ANDERSON N. LEIGH ET AL: "Multiplexed measurement of protein biomarkers in high-frequency longitudinal dried blood spot (DBS) samples: characterization of inflammatory responses", BIORXIV, 20 May 2019 (2019-05-20), XP055836736, Retrieved from the Internet <URL:https://www.biorxiv.org/content/10.1101/643239v1.full.pdf> [retrieved on 20210901], DOI: 10.1101/643239
- ZAKARIA ROSITA ET AL: "eJIFCC2016Vol27No4pp288-317 In this issue: Recent Developments in the Clinical Application of Mass Spectrometry Advantages and challenges of dried blood spot analysis by mass spectrometry across the total testing process", EJIFCC, 1 December 2016 (2016-12-01), pages 288 - 317, XP055836787, Retrieved from the Internet <URL:https://www.ncbi.nlm.nih.gov/pmc/articles/PMC5282914/pdf/ejifcc-27-288.pdf> [retrieved on 20210901]
- ELLUL SUSAN ET AL: "Sex differences in infant blood metabolite profile in association with weight and adiposity measures", PEDIATRIC RESEARCH, LIPPINCOTT WILLIAMS & WILKINS, NEW YORK, US, vol. 88, no. 3, 17 January 2020 (2020-01-17), pages 473 - 483, XP037231318, ISSN: 0031-3998, [retrieved on 20200117], DOI: 10.1038/S41390-020-0762-4
- WHITE SARA L ET AL: "Metabolic profiling of gestational diabetes in obese women during pregnancy", DIABETOLOGIA, SPRINGER, BERLIN, DE, vol. 60, no. 10, 1 August 2017 (2017-08-01), pages 1903 - 1912, XP036309456, ISSN: 0012-186X, [retrieved on 20170801], DOI: 10.1007/S00125-017-4380-6
- RITCHIE SCOTT C. ET AL: "The Biomarker GlycA Is Associated with Chronic Inflammation and Predicts Long-Term Risk of Severe Infection", CELL SYSTEMS, vol. 1, no. 4, 1 October 2015 (2015-10-01), US, pages 293 - 301, XP055837905, ISSN: 2405-4712, Retrieved from the Internet <URL:https://www.cell.com/cell-systems/pdf/S2405-4712(15)00145-3.pdf> DOI: 10.1016/j.cels.2015.09.007
- RIVAL THOMAS ET AL: "Alteration of plasma phospholipid fatty acid profile in patients with septic shock", BIOCHIMIE, MASSON, PARIS, FR, vol. 95, no. 11, 15 August 2013 (2013-08-15), pages 2177 - 2181, XP028731048, ISSN: 0300-9084, DOI: 10.1016/J.BIOCHI.2013.08.006
- NOVAK FRANTISEK ET AL: "Plasma Phospholipid Fatty Acid Profile is Altered in Both Septic and Non-Septic Critically Ill: A Correlation with Inflammatory Markers and Albumin", LIPIDS, SPRINGER, DE, vol. 52, no. 3, 30 December 2016 (2016-12-30), pages 245 - 254, XP036185369, ISSN: 0024-4201, [retrieved on 20161230], DOI: 10.1007/S11745-016-4226-X
- ZHENG YALI ET AL: "Inflammatory responses relate to distinct bronchoalveolar lavage lipidome in community-acquired pneumonia patients: a pilot study", RESPIRATORY RESEARCH, vol. 20, no. 1, 1 December 2019 (2019-12-01), XP055860510, Retrieved from the Internet <URL:https://respiratory-research.biomedcentral.com/track/pdf/10.1186/s12931-019-1028-8.pdf> DOI: 10.1186/s12931-019-1028-8
- GUPTA RUBY ET AL: "Use of dried blood for measurement of trans fatty acids", NUTRITION JOURNAL, BIOMED CENTRAL, GB, vol. 8, no. 1, 24 July 2009 (2009-07-24), pages 35, XP021058628, ISSN: 1475-2891, DOI: 10.1186/1475-2891-8-35

## Description

### TECHNICAL FIELD

The present disclosure relates generally to methods for determining whether a subject has a disease or condition or is at risk of developing a disease or condition. The scope of protection is defined by the appended claims.

### BACKGROUND

Various biomarkers may be useful for predicting whether a subject has a particular disease or condition or is at risk of developing the disease or condition. Such biomarkers may be measured from various biological samples, such as from blood samples or other biological fluids. However, certain sample types may be more complex than others when quantitative measurements of biomarkers are to be obtained. There may also be a need for biomarkers that are predictive for e.g. severe diseases causing hospitalization or even death, such as severe infectious diseases and their complications, for example sepsis, pneumonia and other lower respiratory infections. Ritchie Scott C. et al.: "The Biomarker GlycA Is Associated with Chronic Inflammation and Predicts Long-Term Risk of Severe Infection",CELL SYSTEMS, vol. 1, no. 4, 1 October 2015 (2015-10-01), pages 293-301, reviews glycoprotein acetylation as a biomarker.

### SUMMARY

A method for determining whether a subject has a disease or condition or is at risk of developing a disease or condition is disclosed. The method may comprise
determining in a biological sample obtained from the subject a quantitative value of at least one biomarker relative to a quantitative value of albumin in the biological sample; and
comparing the quantitative value(s) of the at least one biomarker to a control sample or to a control value;
wherein an increase or a decrease in the quantitative value(s) of the at least one biomarker, when compared to the control sample or to the control value, is/are indicative of the subject having the disease or condition or having an increased risk of developing the disease or condition.

A method for determining whether a subject is at risk of developing an infectious disease or a complication thereof is also disclosed. The method may comprise
determining in a biological sample obtained from the subject a quantitative value of at least one biomarker of the following:
   - glycoprotein acetyls,
   - albumin,
   - omega-6 fatty acids,
   - monounsaturated fatty acids,
   - saturated fatty acids,
   - omega-3 fatty acids,
   - apolipoprotein A1 (ApoA1),
   - docosahexaenoic acid (DHA),
   - leucine,
   - acetate,
   - alanine,
   - apolipoprotein B (ApoB),
   - glutamine,
   - isoleucine,
   - linoleic acid,
   - phenylalanine,
   - tyrosine,
   - degree of unsaturation of fatty acids,
   - valine,
   - histidine,
   - cholesterol in HDL (HDL-C),
   - glucose,
   - acetoacetate,
   - 3-hydroxybutyrate,
   - cholesterol in LDL (LDL-C),
   - lactate,
   - triglycerides in LDL (LDL-TG),
   - pyruvate, or
   - cholesterol in VLDL (VLDL-C); and
comparing the quantitative value(s) of the at least one biomarker to a control sample or to a control value;
wherein an increase or a decrease in the quantitative value(s) of the at least one biomarker, when compared to the control sample or to the control value, is/are indicative of the subject having an increased risk of developing the infectious disease or the complication thereof. Claimed is a method for determining whether a subject has a disease or condition or is at risk of developing a disease or condition, wherein the method comprises determining in a biological sample obtained from the subject a quantitative value of at least one biomarker relative to a quantitative value of albumin in the biological sample; and comparing the quantitative value(s) of the at least one biomarker to a control sample or to a control value; wherein an increase or a decrease in the quantitative value (s) of the at least one biomarker, when compared to the control sample or to the control value, is/are indicative of the subject having the disease or condition or having an increased risk of developing the disease or condition; wherein the biological sample is a dry blood sample, a dry sample obtainable from blood, or obtainable from a dry blood sample; wherein the quantitative value of the at least one biomarker and the quantitative value of the albumin are measured using nuclear magnetic spectroscopy; and wherein the at least one biomarker comprises or is glycoprotein acetyls.

### BRIEF DESCRIPTION OF THE DRAWINGS

The accompanying drawings, which are included to provide a further understanding of the embodiments and constitute a part of this specification, illustrate various embodiments. In the drawings:
**Figure 1a** shows the relation of baseline biomarker levels to future development of type 2 diabetes, when the biomarkers are analysed in absolute concentrations and when scaled relative to the concentration of albumin.
**Figure 1b** shows the relation of baseline biomarker levels to future development of severe pneumonia, when the biomarkers are analysed in absolute concentrations and when scaled relative to the concentration of albumin.
**Figure 2a** shows the relation of baseline biomarker levels to future development of pneumonia (severe or non-severe), when the biomarker concentrations are analysed in absolute concentrations.
**Figure 2b** shows the relation of baseline biomarker levels to future development of Other Lower Respiratory Diseases (acute bronchitis, acute bronchiolitis, and Unspecified acute lower respiratory infection), when the biomarker concentrations are analysed in absolute concentrations.
**Figure 2c** shows the relation of baseline biomarker levels to future development of sepsis, when the biomarker concentrations are analysed in absolute concentrations.
**Figure 3a** shows the relation of baseline biomarker levels for 3 plurality-biomarker-scores to future development of severe pneumonia for the whole study population and in the following subgroups of individuals: study participants without chronic respiratory or cardiometabolic disease at baseline, in different age groups (divided in age-tertiles of the study population), and separately for men and women. The relation of the of baseline biomarker levels for 3 different plurality-biomarker-scores with severe pneumonia occurring within 2 years after the blood samples were taken is also shown.

### DETAILED DESCRIPTION

In one aspect, a method for determining whether a subject has a disease or condition or is at risk of developing a disease or condition is disclosed.

The method may comprise determining in a biological sample obtained from the subject a quantitative value of at least one biomarker relative to a quantitative value of albumin in the biological sample; and
comparing the quantitative value(s) of the at least one biomarker to a control sample or to a control value;
wherein an increase or a decrease in the quantitative value(s) of the at least one biomarker, when compared to the control sample or to the control value, is/are indicative of the subject having the disease or condition or having an increased risk of developing the disease or condition.

In a second aspect, a method for determining whether a subject is at risk of developing an infectious disease or a complication thereof is disclosed. The method may comprise
determining in a biological sample obtained from the subject a quantitative value of at least one biomarker (or a plurality of the biomarkers, for example at least two, or at least three, or at least four, or at least five, or at least six, or at least seven, or at least eight, or at least nine, or at least 10, or all) of the following:
   - glycoprotein acetyls,
   - albumin,
   - omega-6 fatty acids,
   - monounsaturated fatty acids,
   - saturated fatty acids,
   - omega-3 fatty acids,
   - apolipoprotein A1 (ApoA1),
   - docosahexaenoic acid (DHA),
   - leucine,
   - acetate,
   - alanine,
   - apolipoprotein B (ApoB),
   - glutamine,
   - isoleucine,
   - linoleic acid,
   - phenylalanine,
   - tyrosine,
   - degree of unsaturation of fatty acids,
   - valine,
   - histidine,
   - cholesterol in HDL (HDL-C),
   - glucose,
   - acetoacetate,
   - 3-hydroxybutyrate,
   - cholesterol in LDL (LDL-C),
   - lactate,
   - triglycerides in LDL (LDL-TG),
   - pyruvate, or
   - cholesterol in VLDL (VLDL-C); and
comparing the quantitative value(s) of the at least one biomarker to a control sample or to a control value;
wherein an increase or a decrease in the quantitative value(s) of the at least one biomarker, when compared to the control sample or to the control value, is/are indicative of the subject having an increased risk of developing the infectious disease or the complication thereof.

Any embodiments described below may be understood as relating to either one (or both) of the above aspects. Hereinafter, the terminology "embodiment" relates to the disclosure and only forms part of the invention if it falls within the scope of the claims.

The biological sample may be a dry blood sample, a dry sample obtainable from blood, or a biological sample obtainable from a dry blood sample. However, in the context of this specification, the biological sample does not necessarily have to be a dry blood sample or obtainable from a dry blood sample, as will be set out in more detail below.

The dry blood sample may be a dry whole blood sample. However, the term may be understood, at least in some embodiments, to cover also dry samples containing serum and/or plasma (but not necessarily whole blood). The term "dry blood sample" or "dry sample obtainable from blood" may, in some embodiments, refer to a dry sample obtainable from separated blood.

The dry blood sample or the dry sample obtainable from blood may be obtainable from a fingertip of the subject, sampling of capillary blood from the upper arm of the subject, and/or by venepuncture of the subject. However, they may be additional or alternative locations and/or methods of obtaining the blood.

The method may comprise determining in the biological sample quantitative values of a plurality of biomarkers. Thus any references to "at least one biomarker" in this specification may also be understood as referring to a plurality of biomarkers. For example, the plurality of the biomarkers may comprise 2, 3, 4, 5, 6, 7, 8, 9, 10, or more (i.e. at least 2, at least 3, at least 4, at least 5, at least 6, at least 7, at least 8, at least 9, or at least 10) biomarkers. The term "plurality of the biomarkers" may thus, within this specification, be understood as referring to any number (above one) of biomarkers. The term "plurality of the biomarkers" may thus be understood as referring to any number (above one) and/or combination or subset of the biomarkers described in this specification. Determining a plurality of biomarkers may increase the accuracy of the prediction of whether the subject has or is at risk of developing a disease or condition. In general, it may be that the larger the number of the biomarkers, the more accurate or predictive the method. The plurality of biomarkers may be measured from the same biological sample or from separate biological samples and using the same analytical method or different analytical methods. In an embodiment, the plurality of biomarkers may be a panel of a plurality of biomarkers.

At least in an embodiment, in the context of this specification, the wording "comparing the quantitative value(s) of the biomarker(s) to a control sample or to a control value(s)" may be understood, as a skilled person would, as referring to comparing the quantitative value or values of the biomarker or biomarkers, to a control sample or to a control value(s) either individually or as a plurality of biomarkers (e.g. when a risk score is calculated from the quantitative values of a plurality of biomarkers), depending e.g. on whether the quantitative value of a single (individual) biomarker or the quantitative values of a plurality of biomarkers are determined.

In an embodiment, the method comprises determining in a biological sample obtained from the subject a quantitative value of at least one biomarker (or a plurality of the biomarkers, for example at least two, or at least three, or at least four, or at least five, or at least six, or at least seven, or at least eight, or at least nine, or at least 10, or all) of the following relative to the quantitative value of the albumin in the biological sample:
- glycoprotein acetyls,
- omega-6 fatty acids,
- monounsaturated fatty acids,
- saturated fatty acids,
- omega-3 fatty acids,
- apolipoprotein A1 (ApoA1),
- docosahexaenoic acid (DHA),
- leucine,
- acetate,
- alanine,
- apolipoprotein B (ApoB),
- glutamine,
- isoleucine,
- linoleic acid,
- phenylalanine,
- tyrosine,
- degree of unsaturation of fatty acids,
- valine,
- histidine,
- cholesterol in HDL (HDL-C),
- glucose,
- acetoacetate,
- 3-hydroxybutyrate,
- cholesterol in LDL (LDL-C),
- lactate,
- triglycerides in LDL (LDL-TG),
- pyruvate, or
- cholesterol in VLDL (VLDL-C).

In an embodiment, the method comprises determining a quantitative value of glycoprotein acetyls.

In an embodiment, the method comprises determining a quantitative value of albumin.

In an embodiment, the method comprises determining a quantitative value of omega-6 fatty acids.

In an embodiment, the method comprises determining a quantitative value of monounsaturated fatty acids.

In an embodiment, the method comprises determining a quantitative value of saturated fatty acids.

In an embodiment, the method comprises determining a quantitative value of omega-3 fatty acids.

In an embodiment, the method comprises determining a quantitative value of apolipoprotein A1 (ApoA1).

In an embodiment, the method comprises determining a quantitative value of docosahexaenoic acid (DHA).

In an embodiment, the method comprises determining a quantitative value of leucine.

In an embodiment, the method comprises determining a quantitative value of acetate.

In an embodiment, the method comprises determining a quantitative value of alanine.

In an embodiment, the method comprises determining a quantitative value of apolipoprotein B (ApoB).

In an embodiment, the method comprises determining a quantitative value of glutamine.

In an embodiment, the method comprises determining a quantitative value of isoleucine.

In an embodiment, the method comprises determining a quantitative value of linoleic acid.

In an embodiment, the method comprises determining a quantitative value of phenylalanine.

In an embodiment, the method comprises determining a quantitative value of tyrosine.

In an embodiment, the method comprises determining a quantitative value of degree of unsaturation of fatty acids.

In an embodiment, the method comprises determining a quantitative value of valine.

In an embodiment, the method comprises determining a quantitative value of histidine.

In an embodiment, the method comprises determining a quantitative value of cholesterol in HDL (HDL-C).

In an embodiment, the method comprises determining a quantitative value of glucose.

In an embodiment, the method comprises determining a quantitative value of acetoacetate.

In an embodiment, the method comprises determining a quantitative value of 3-hydroxybutyrate.

In an embodiment, the method comprises determining a quantitative value of cholesterol in LDL (LDL-C).

In an embodiment, the method comprises determining a quantitative value of lactate.

In an embodiment, the method comprises determining a quantitative value of triglycerides in LDL (LDL-TG).

In an embodiment, the method comprises determining a quantitative value of pyruvate.

In an embodiment, the method comprises determining a quantitative value of cholesterol in VLDL (VLDL-C).

In an embodiment, the method comprises determining in a biological sample obtained from the subject a quantitative value of at least one biomarker (or a plurality of the biomarkers, for example at least two, or at least three, or at least four, or all) of the following relative to the quantitative value of the albumin in the biological sample:
- omega-6 fatty acids,
- monounsaturated fatty acids,
- saturated fatty acids, or
- omega-3 fatty acids.

In an embodiment, the method comprises determining in a biological sample obtained from the subject a quantitative value of at least one biomarker (or a plurality of the biomarkers, for example at least two, or at least three, or at least four, or at least five, or at least six, or at least seven, or at least eight, or at least nine, or at least 10, or at least 11, or at least 12, or at least 13, or at least 14, or all) of the following relative to the quantitative value of the albumin in the biological sample:
- apolipoprotein A1 (ApoA1),
- docosahexaenoic acid (DHA),
- leucine,
- acetate,
- alanine,
- apolipoprotein B (ApoB),
- glutamine,
- isoleucine,
- linoleic acid,
- phenylalanine,
- tyrosine,
- degree of unsaturation of fatty acids,
- valine, or
- histidine.

In an embodiment, the method comprises determining in a biological sample obtained from the subject a quantitative value of at least one biomarker (or a plurality of the biomarkers, for example at least two, or at least three, or at least four, or at least five, or at least six, or at least seven, or at least eight, or at least nine, or all) of the following relative to the quantitative value of the albumin in the biological sample:
- cholesterol in HDL (HDL-C),
- glucose,
- acetoacetate,
- 3-hydroxybutyrate,
- cholesterol in LDL (LDL-C),
- lactate,
- triglycerides in LDL (LDL-TG),
- pyruvate, or
- cholesterol in VLDL (VLDL-C).

In an embodiment, in particular of the second aspect, the method comprises
determining in the biological sample obtained from the subject a quantitative value of glycoprotein acetyls; and/or
determining in the biological sample obtained from the subject a quantitative value of at least one biomarker of the following:
   - omega-6 fatty acids,
   - monounsaturated fatty acids,
   - saturated fatty acids, or
   - omega-3 fatty acids; and/or
determining in the biological sample obtained from the subject a quantitative value of at least one biomarker of the following:
   - apolipoprotein A1 (ApoA1),
   - docosahexaenoic acid (DHA),
   - leucine,
   - acetate,
   - alanine,
   - apolipoprotein B (ApoB),
   - glutamine,
   - isoleucine,
   - linoleic acid,
   - phenylalanine,
   - tyrosine,
   - degree of unsaturation of fatty acids,
   - valine, or
   - histidine; and/or
determining in the biological sample obtained from the subject a quantitative value of at least one biomarker of the following:
   - cholesterol in HDL (HDL-C),
   - glucose,
   - acetoacetate,
   - 3-hydroxybutyrate,
   - cholesterol in LDL (LDL-C),
   - lactate,
   - triglycerides in LDL (LDL-TG),
   - pyruvate, or
   - cholesterol in VLDL (VLDL-C).

The subject may be human. The subject may, additionally or alternatively, be an animal, such as a mammal, for example, a non-human primate, a dog, a cat, a horse, a sheep, a goat, a bovine, a rabbit, a pig and/or a rodent, such as a mouse or a rat, or any other species.

In the context of this specification, the term "biomarker" may refer to a biomarker, for example a chemical or molecular marker, that may be found associated with a disease or a condition or the risk of having or developing thereof. It does not necessarily refer to a biomarker that would be statistically fully validated as having a specific effectiveness in a clinical setting. The biomarker may be a metabolite, a compound, a lipid, a protein, a moiety, a functional group, a composition, a combination of two or more metabolites and/or compounds, a (measurable or measured) quantity thereof, a ratio or other value derived thereof, or in principle any measurement reflecting a chemical and/or biological component that may be found associated with a disease or condition or the risk of having or developing thereof. The biomarkers and any combinations thereof, optionally in combination with further measures, may be used to indicate or measure a biological process specific to and/or indicative of a specific disease or condition or the risk of developing the same, such as e.g. an infectious disease or a complication thereof, sepsis, pneumonia, other lower respiratory infection, or diabetes. Any "ratio" of two biomarkers or of at least one biomarker to albumin may refer to the ratio of the quantitative values of the biomarkers; or the ratio of the quantitative value of the biomarker and the quantitative value of the albumin; to a ratio of a quantitative value of a biomarker to another measure (e.g. a quantitative value of a metabolite or compound, or total quantitative value of metabolites or compounds of the same class); or to any combination thereof.

When the quantitative value of the at least one biomarker is determined relative to the quantitative value of the albumin in the biological sample, it may be possible to obtain relatively accurate quantitative value for the at least one biomarker. In other words, the (initial) quantitative value of the at least one biomarker (or the (initial) quantitative values of the plurality of biomarkers) may be normalized against or scaled to the quantitative value of the albumin. This may be possible also in cases in which there may be dilution effects, for example when using a dry blood sample that is subsequently diluted prior to determining the quantitative value of the at least one biomarker, and/or when the dry blood sample or other biological sample is obtainable such that the source of the sample may cause dilution effects, for example when using a blood sample obtainable from a fingertip of the subject. Fingertip samples may contain additional fluids, such as tissue fluid, in addition to the blood drawn from the fingertip, the relative volume of which may be challenging to control.

Therefore, it may be possible to accurately measure the at least one biomarker from dry samples, such as dry blood samples. The ratio of such measures may be considered to be in good correspondence to the values obtained from venous blood.

However, in some embodiments, in particular certain embodiments according to the second aspect, it may be possible to determine a quantitative value of at least one biomarker relative to a quantitative value of a scaling biomarker (other than albumin) in the biological sample. Alternatively or additionally, certain other markers may also be useful for normalizing or scaling the quantitative value of the at least one biomarker.

The quantitative value of the at least one biomarker relative to the quantitative value of the albumin in the biological sample may be determined, for example, by calculating a ratio of an initial quantitative value of the at least one biomarker and the quantitative value of the albumin in the biological sample. It may also be possible to use an additional mathematical transformation, for example for presentation of the results, e.g. to the subject.

The method may further comprise determining whether the subject has the disease or condition or is at risk of developing the disease or condition using a risk score, hazard ratio, and/or predicted absolute risk calculated on the basis of the quantitative value(s) of the at least one biomarker or of the plurality of the biomarkers relative to the quantitative value of the albumin.

The risk score and/or hazard ratio and/or predicted absolute risk may be calculated based on any plurality, combination or subset of biomarkers described in this specification.

The risk score and/or hazard ratio and/or predicted absolute risk may be calculated e.g. as shown in the Examples below. For example, the plurality of biomarkers measured using a suitable method, for example with NMR spectroscopy, may be combined using regression algorithms and multivariate analyses and/or using machine learning analysis. Before regression analysis or machine learning, any missing values in the biomarkers may be imputed with the mean value of each biomarker for the dataset. A number of biomarkers, for example ten, that may be considered most associated with the onset of the disease or condition may be selected for use in the prediction model. Other modelling approaches may be used to calculate a risk score and/or hazard ratio and/or predicted absolute risk based on a subset of individual biomarkers, i.e. a plurality of the biomarkers.

An increase or a decrease in the risk score, hazard ratio, odds ratio, and/or predicted absolute risk and/or relative risk may be indicative of the subject having an increased risk of developing the disease or condition.

In the context of this specification, the term "glycoprotein acetyls", "glycoprotein acetylation", or "GlycA" may refer to the abundance of circulating glycated proteins, and/or to a nuclear magnetic resonance spectroscopy (NMR) signal that represents the abundance of circulating glycated proteins, i.e. N-acetylated glycoproteins. Glycoprotein acetyls may include signals from a plurality of different glycoproteins, including e.g. alpha-1-acid glycoprotein, alpha-1 antitrypsin, haptoglobin, transferrin, and/or alpha-1 antichymotrypsin. Glycoprotein acetyls and a method for measuring them is described e.g. in Kettunen et al., 2018, Circ Genom Precis Med. 11:e002234 and Soininen et al., 2009, Analyst 134, 1781-1785.

In the context of this specification, the term "albumin" may be understood as referring to serum albumin (often referred to as blood albumin). It is an albumin found in vertebrate blood. For example, human albumin (human serum albumin) is encoded by the *ALB* gene. Serum albumin is the most abundant blood protein in mammals. Albumin is a globular, watersoluble, un-glycosylated serum protein of approximate molecular weight of 65,000 Daltons. The measurement of albumin using NMR is described e.g. in publications by Kettunen et al., 2012, Nature Genetics 44, 269-276; Soininen et al., 2015, Circulation: Cardiovascular Genetics 8, 192-206 (DOI: 10.1161/CIRCGENETICS.114.000216) and Würtz et al., 2017, American Journal of Epidemiology 186 (9), 1084-1096 (DOI: 10.1093/aje/kwx016). Albumin may also be measured by various other methods, for example in a clinical setting. Examples of such methods may include e.g. dye-binding methods such as bromocresol green and bromocresol purple.

In the context of this specification, the term "HDL" refers to high-density lipoprotein.

In the context of this specification, the term "LDL" refers to low-density lipoprotein.

In the context of this specification, the term "VLDL" refers to very-low-density lipoprotein.

In the context of this specification, the term "omega-6 fatty acids" may refer to total omega-6 fatty acids. Omega-6 fatty acids are polyunsaturated fatty acids. In omega-6 fatty acids, the last double bond in the fatty acid chain is the sixth bond counting from the methyl end.

In the context of this specification, the term "monounsaturated fatty acids" (MUFAs) may refer to total monounsaturated fatty acids. Monounsaturated fatty acids have one double bond in their fatty acid chain. The MUFAs may (mainly) include omega-9 and omega-7 fatty acids. Oleic acid (18:1ω-9), palmitoleic acid (16:1ω-7) and cis-vaccenic acid (18:1ω-7) are examples of common MUFAs in human serum.

In the context of this specification, the term "saturated fatty acids" (SFAs) may refer to total saturated fatty acids. Saturated fatty acids may be or comprise fatty acids which have no double bonds in their structure. Palmitic acid (16:0) and stearic acid (18:0) are examples of abundant SFAs in human serum.

In the context of this specification, the term "omega-3 fatty acids" may refer to total omega-3 fatty acids. Omega-3 fatty acids are polyunsaturated fatty acids. In omega-3 fatty acids, the last double bond in the fatty acid chain is the third bond counting from the methyl end.

For any or all fatty acid measures, including omega-3, DHA, LA, MUFA, SFA, the fatty acid measures, for example the total omega-6-fatty acids, include blood (or serum/plasma) free fatty acids, bound fatty acids and esterified fatty acids. Esterified fatty acids may, for example, be esterified to glycerol as in triglycerides, diglycerides, monoglycerides, or phosphoglycerides, or to cholesterol as in cholesterol esters.

In the context of this specification, the term "apolipoprotein(s)" may refer to apolipoprotein molecules which are amphipathic proteins and key structural components in the surface area of lipoprotein particles. They may include apolipoprotein B (ApoB) and apolipoprotein A1 (ApoA1).

In the context of this specification, the term "degree of unsaturation of fatty acids" may be understood as referring to the number of double bonds in total fatty acids, for example the average number of double bonds in total fatty acids.

In the context of this specification, the phrase "cholesterol in HDL", or cholesterol in any other lipoprotein class, such as in LDL or VLDL, may be understood as referring to total cholesterol in said lipoprotein class or subfraction.

In the context of this specification, the term "quantitative value" may refer to any quantitative value characterizing the amount and/or concentration of a biomarker. For example, it may be the amount or concentration of the biomarker in the biological sample, or it may be a signal derived from nuclear magnetic spectroscopy (NMR) or other method suitable for detecting the biomarker in a quantitative manner. Such a signal may be indicative of or may correlate with the amount or concentration of the biomarker. It may also be a quantitative value calculated from one or more signals derived from NMR measurements or from other measurements. Quantitative values may, additionally or alternatively, be measured using a variety of techniques. Such methods may include mass spectrometry (MS), gas chromatography combined with MS, high performance liquid chromatography alone or combined with MS, immunoturbidimetric measurements, ultracentrifugation, ion mobility, enzymatic analyses, colorimetric or fluorometric analyses, immunoblot analysis, immunohistochemical methods (e.g. *in situ* methods based on antibody detection of metabolites), and immunoassays (e.g. ELISA). Examples of various methods are set out below. The method used to determine the quantitative value(s) in the subject should be the same method that is used to determine the quantitative value(s) in a control subject/control subjects or in a control sample/control samples.

In the context of this specification, the term "quantitative value of albumin" may refer to any quantitative value characterizing the amount and/or concentration of the albumin. For example, it may be the amount or concentration of the albumin in the biological sample, or it may be a signal derived from nuclear magnetic spectroscopy (NMR) or other method suitable for detecting the albumin in a quantitative manner. Such a signal may be indicative of or may correlate with the amount or concentration of the albumin. It may also be a quantitative value calculated from one or more signals derived from NMR measurements or from other measurements. Quantitative values may, additionally or alternatively, be measured using a variety of techniques. Such methods may include mass spectrometry (MS), gas chromatography combined with MS, high performance liquid chromatography alone or combined with MS, immunoturbidimetric measurements, ultracentrifugation, ion mobility, enzymatic analyses, colorimetric or fluorometric analyses, immunoblot analysis, immunohistochemical methods (e.g. *in situ* methods based on antibody detection), and immunoassays (e.g. ELISA). Examples of various methods are set out below. The method used to determine the quantitative value(s) in the subject should be the same method that is used to determine the quantitative value(s) in a control subject/control subjects or in a control sample/control samples.

In the context of this specification, the term "control subject" may refer to a subject known not to suffer from the disease or condition, and/or known not to be at risk of having or developing the disease or condition. The control subject may be a matched control subject.

The disease may be an infectious disease or a complication thereof.

In an embodiment, the disease is a severe infectious disease, a severe infection or a (severe) complication thereof.

In an embodiment, the infectious disease or the complication thereof is sepsis.

In an embodiment, the infectious disease or the complication thereof is pneumonia.

In an embodiment, the infectious disease or the complication thereof is other lower respiratory disease (i.e. a lower respiratory tract disease).

In the context of this specification, the term "pneumonia" may be understood as referring to inflammation of one or both lungs of the subject. It may be caused e.g. by a bacterial, fungal and/or viral infection. The infection may involve inflammation of the air sacs in one or both lungs of the subject. The signs and symptoms of pneumonia may vary from mild to severe or potentially life-threatening, depending on factors such as the type of microorganism causing the infection, age and/or overall health of the subject. Pneumonia is the leading cause of death from infectious diseases worldwide.

In an embodiment, the pneumonia is severe pneumonia. Severe pneumonia may include or result in e.g. hospitalization and/or death.

In an embodiment, the method comprises determining a quantitative value of glycoprotein acetyls.

Glycoprotein acetyls may be useful for determining whether the subject is at risk of developing an infectious disease or a complication thereof, for example sepsis, pneumonia, such as severe pneumonia, or other lower respiratory infection.

In an embodiment, the disease or complication thereof is diabetes, such as type 2 diabetes.

The method may further comprise determining whether the subject is at risk of developing the infectious disease or the complication thereof using a risk score, hazard ratio, and/or predicted absolute risk calculated on the basis of the quantitative value(s) of the at least one biomarker or of the plurality of the biomarkers.

The risk score, hazard ratio, and/or predicted absolute risk may be calculated based on any plurality, combination or subset of biomarkers described in this specification.

The risk score, hazard ratio, and/or predicted absolute risk may be calculated e.g. as shown in the Examples below. For example, the plurality of biomarkers measured using a suitable method, for example with NMR spectroscopy, may be combined using regression algorithms and multivariate analyses and/or using machine learning analysis. Before regression analysis or machine learning, any missing values in the biomarkers may be imputed with the mean value of each biomarker for the dataset. A number of biomarkers, for example ten, that may be considered most associated with the onset of the disease or condition may be selected for use in the prediction model. Other modelling approaches may be used to calculate a risk score and/or hazard ratio and/or predicted absolute risk based on a subset of individual biomarkers, i.e. a plurality of the biomarkers.

In an embodiment, a method for determining whether a subject has pneumonia or is at risk of developing pneumonia comprises
determining in a biological sample obtained from the subject a quantitative value of at least one biomarker relative to a quantitative value of albumin in the biological sample; and
comparing the quantitative value(s) of the at least one biomarker to a control sample or to a control value;
wherein an increase or a decrease in the quantitative value(s) of the at least one biomarker, when compared to the control sample or to the control value, is/are indicative of the subject having pneumonia or having an increased risk of developing pneumonia.

In an embodiment of the above embodiment, the at least one biomarker may comprise or be glycoprotein acetyls.

In an embodiment of the above aspect, the biological sample may be a dry blood sample or obtainable from a dry blood sample. However, the biological sample may, alternatively or additionally, be any other biological sample, for example any biological sample described in this specification.

The quantitative value, or the initial quantitative value, of the at least one biomarker, or the plurality of the biomarkers, and/or the quantitative value of the albumin may be measured using nuclear magnetic spectroscopy (NMR), for example ¹H-NMR. The at least one additional biomarker, or the plurality of the additional biomarkers, may also be measured using NMR. NMR may provide a particularly efficient and fast way to measure biomarkers, including a large number of biomarkers simultaneously, and can provide quantitative values for them. NMR also typically requires very little sample pre-treatment or preparation. The biomarkers measured with NMR can effectively be measured for large amounts of samples using an assay for blood (serum or plasma) NMR metabolomics previously published by Soininen et al., 2015, Circulation: Cardiovascular Genetics 8, 192-206 (DOI: 10.1161/CIRCGENETICS.114.000216); Soininen et al., 2009, Analyst 134, 1781-1785; and Würtz et al., 2017, American Journal of Epidemiology 186 (9), 1084-1096 (DOI: 10.1093/aje/kwx016). This provides data on 228 biomarkers per sample as described in detail in the above scientific papers.

In an embodiment, the (initial) quantitative value of the at least one biomarker and/or the quantitative value of the albumin is/are measured using nuclear magnetic spectroscopy.

However, quantitative values for various biomarkers described in this specification may also be performed by techniques other than NMR. For example, mass spectrometry (MS), enzymatic methods, antibody-based detection methods, or other biochemical or chemical methods may be contemplated, depending on the biomarker.

For example, glycoprotein acetyls can be measured or approximated by immunoturbidimetric measurements of alpha-1-acid glycoprotein, haptoglobin, alpha-1-antitrypsin, and transferrin (e.g. as described in Ritchie et al., 2015, Cell Syst. 28;1(4) :293-301).

E.g. monounsaturated fatty acids and polyunsaturated fatty acids can be quantified (i.e. their quantitative values may be determined) by serum total fatty acid composition using gas chromatography (for example, as described in Jula et al., 2005, Arterioscler Thromb Vasc Biol 25, 1952-1959).

Cholesterol in lipoprotein fractions can be quantified using high performance liquid chromatography.

Apolipoprotein B, apolipoprotein A1, and glucose can be quantified by enzymatic clinical chemistry analysers, such as ROCHE COBAS 6000.

In the context of this specification, the term "sample" or "biological sample" may refer to any biological sample obtained from a subject or a group or population of subjects. The sample may be fresh, frozen, or dry.

The biological sample (in particular in embodiments in which the biological sample is not a dry blood sample) may comprise or be, for example, a blood sample, a plasma sample, a serum sample, or a sample derived therefrom. The biological sample may be, for example, a fasting blood sample, a fasting plasma sample, a fasting serum sample, or a fraction obtainable therefrom. However, the biological sample does not necessarily have to be a fasting sample. The blood sample may be a venous blood sample.

The dry blood sample may be a dried whole blood sample, a dried plasma sample, a dried serum sample, or a dried sample derived therefrom.

Some of the biomarkers may be determined from body fluids other than blood or fractions obtainable therefrom. The biological sample may, additionally or alternatively, comprise or be a sample or one or more other body fluids or biofluids, for example an amniotic fluid sample, a urine sample, a saliva sample, a bile sample, a tear sample and/or a spinal fluid sample.

The method may comprise obtaining the biological sample from the subject prior to determining the quantitative value of the at least one biomarker. Taking a blood sample or a tissue sample of a subject or patient is a part of normal clinical practice. The collected blood or tissue sample can be prepared and serum or plasma can be separated using techniques well known to a skilled person. Methods for separating one or more fractions from biological samples, such as blood samples or tissue samples, are also available to a skilled person. The term "fraction" may, in the context of this specification, also refer to a portion or a component of the biological sample separated according to one or more physical properties, for instance solubility, hydrophilicity or hydrophobicity, density, or molecular size.

The dry blood sample may be, in principle, any dried blood sample.

The dry blood sample may be a dried blood spot. The dry blood sample, for example for NMR analysis, may be provided as dried blood spot on filter paper or other substrate or media that is capable of absorbing blood. Examples of other substrates or media may comprise or be e.g. various fibers and/or resins. The steps related to providing the sample and applying blood, which can be for example obtained either with venepuncture or fingerprick, on a paper, e.g. a filter paper, or other substrate or media that can absorb blood; and drying the blood on the paper or other substrate or media where the blood is applied. During the drying process, the blood may also be passively separated to its components, for example to blood cells and plasma or serum, on the paper or other media where the blood is applied. The piece of the paper or other media where the blood or its components have dried may be punched or otherwise cut out of the paper or media. Alternatively or additionally, the dry blood sample may be provided as a dry blood sample in a HemaSpot HF device or other suitable device. Various other means for collecting and/or storing dry blood samples may also be contemplated.

The biological sample may be obtainable by further sample preparation from the dry blood sample.

The biological sample may be obtainable from the dry blood sample or from the dry sample obtainable from blood by extracting at least a part of the blood or one or more components thereof from the dry blood sample or from the dry sample obtainable from blood into a solvent. The subsequent sample preparation may include e.g. extracting the blood or its components from the dry blood sample, for example from the cut or punched piece of paper or other media. The blood or its components may be extracted with e.g. an aqueous buffer, or other suitable solvent, thus providing a liquid sample for NMR analysis or other suitable analytical method. A deuterated solvent may be added for ¹H NMR.

In the context of this specification, the term "control sample" may refer to a sample obtained from a subject and known not to suffer from the disease or condition or not being at risk of having or developing the disease or condition. The control sample may be matched. In an embodiment, the control sample may be a biological sample from a healthy individual or a generalized population of healthy individuals. The term "control value" may be understood as a value obtainable from the control sample and/or a quantitative value derivable therefrom. For example, it may be possible to calculate a threshold value from control samples and/or control values, above or below which the risk of developing the disease or condition is elevated. In other words, a value higher or lower (depending on the biomarker, risk score, hazard ratio, and/or predicted absolute risk) than the threshold value may be indicative of the subject having an increased risk of developing the disease or condition.

An increase or a decrease in the quantitative value(s) of the at least one biomarker, or the plurality of the biomarkers, when compared to the control sample or to the control value, may be indicative of the subject having an increased risk of having or developing the disease or condition. Whether an increase or a decrease is indicative of the subject having an increased risk of developing the disease or condition, may depend on the biomarker.

A 1.2-fold, 1.5-fold, or for example 2-fold, or 3-fold, increase or a decrease in the quantitative value(s) of the at least one biomarker (or in an individual biomarker of the plurality of the biomarkers) when compared to the control sample or to the control value, may be indicative of the subject having an increased risk of developing the disease or condition.

In an embodiment, an increase in the quantitative value of glycoprotein acetyls, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of glycoprotein acetyls relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of omega-6 fatty acids, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of omega-6 fatty acids relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of monounsaturated fatty acids, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of monounsaturated fatty acids relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of saturated fatty acids, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of saturated fatty acids relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of omega-3 fatty acids, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of omega-3 fatty acids relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of apolipoprotein A1 (ApoA1), when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of apolipoprotein A1 (ApoA1) relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of docosahexaenoic acid (DHA), when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of docosahexaenoic acid (DHA) relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of leucine, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of leucine relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of acetate, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of acetate relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of alanine, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of alanine relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of apolipoprotein B (ApoB), when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of apolipoprotein B (ApoB) relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of glutamine, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of glutamine relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of isoleucine, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of isoleucine relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of linoleic acid (LA), when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of linoleic acid relative to the quantitative value of the albumin in the biological sample , when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of phenylalanine, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of phenylalanine relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of tyrosine, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of tyrosine relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of degree of unsaturation of fatty acids, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of degree of unsaturation of fatty acids relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of valine, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of valine relative to the quantitative value of the albumin in the biological sample , when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of histidine, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of histidine relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of cholesterol in HDL (HDL-C), when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of cholesterol in HDL (HDL-C) relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of glucose, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of glucose relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of acetoacetate, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of acetoacetate relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of 3-hydroxybutyrate, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of 3-hydroxybutyrate relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of cholesterol in LDL (LDL-C), when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of cholesterol in LDL (LDL-C) relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of lactate, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of lactate relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of triglycerides in LDL (LDL-TG), when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of triglycerides in LDL (LDL-TG) relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of pyruvate, when compared to the control sample or to the control value, may be indicative of the subject having an increased risk of developing the infectious disease or the complication thereof, such as pneumonia, other lower respiratory infection, and/or sepsis.

In an embodiment, an increase in the quantitative value of pyruvate relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of cholesterol in VLDL (VLDL-C), when compared to the control sample or to the control value, may be indicative of the subject having an increased risk of developing the infectious disease or the complication thereof, such as pneumonia, other lower respiratory infection, and/or sepsis.

In an embodiment, a decrease in the quantitative value of cholesterol in VLDL (VLDL-C) relative to the quantitative value of the albumin in the biological sample, when compared to the control sample or to the control value, may be indicative of the subject having an increased risk of developing the infectious disease or the complication thereof, such as pneumonia, other lower respiratory infection, and/or sepsis.

### EXAMPLES

Reference will now be made in detail to various embodiments, an example of which is illustrated in the accompanying drawings.

The description below discloses some embodiments in such a detail that a person skilled in the art is able to utilize the embodiments based on the disclosure. Not all steps or features of the embodiments are discussed in detail, as many of the steps or features will be obvious for the person skilled in the art based on this specification.

### EXAMPLE 1

Biomarker measures quantified by nuclear magnetic resonance (NMR) were investigated as to whether they could be predictive of the following infectious diseases and their complications even many years after the blood sampling: Pneumonia and severe Pneumonia, Other lower respiratory diseases, and sepsis. In addition, biomarker associations with type 2 diabetes were examined to explore the effect of scaling the individual biomarker concentrations relative to albumin. All analyses were conducted based on the UK Biobank, with over 100,000 study participants with blood biomarker data from NMR available.

### Study population

Details of the design of the UK Biobank have been reported by Sudlow et al 2015, PLoS Med. 2015;12(3):e1001779. Briefly, UK Biobank recruited 502 639 participants aged 37-73 years in 22 assessment centres across the UK. All participants provided written informed consent and ethical approval was obtained from the North West Multi-Center Research Ethics Committee. Blood samples were drawn at baseline between 2007 and 2010. No selection criteria were applied to the sampling.

### NMR metabolomics

From the entire UK Biobank population, a random subset of baseline plasma samples from 118 466 individuals were measured using the Nightingale high-throughput NMR metabolomics platform. This provides simultaneous quantification of routine lipids, lipoprotein subclass profiling with lipid concentrations within 14 subclasses, fatty acid composition, and various low-molecular weight metabolites including amino acids, ketone bodies and gluconeogenesis-related metabolites in molar concentration units. Technical details and epidemiological applications have been reviewed (Soininen et al 2015, Circ Cardiovasc Genet; 2015;8:192-206; Würtz et al 2017, Am J Epidemiol 2017;186:1084-1096).

The quantified metabolomic biomarker data from UK Biobank samples were curated and approved mid-May 2020. Values outside four interquartile ranges from median were considered as outliers and excluded.

### Analyses of biomarker relations with disease risk

The blood biomarker associations with disease risk were conducted based on UK Biobank data. Analyses focused the relation of biomarker concentration on disease occurrences after the blood samples were collected, to determine how each individual biomarker could predict future disease risk. Examples of how multi-biomarker scores, in the form a weighted plurality of biomarkers, were also explored whether they could be predictive of future disease risk, even stronger than each individual biomarker. Information on disease events occurring after the blood samplings for all study participants were recorded from UK Hospital Episode Statistics data, death registries as well as primary care records and self-reports. All analyses are based on first occurrence of diagnosis, so individuals with recorded diagnosis of the given disease prior to blood sampling were omitted from the statistical analyses. The following diagnoses were used to define the disease endpoints: For type 2 diabetes diagnosis, ICD-10 E11 and operative procedures were used, as described in Rao et al. Circ Genom Precis Med. 2018;11(7):e002162. Self-reported diabetes was not included in the endpoint. Pneumonia-related diagnoses were based on any occurrence of ICD-10 diagnoses J12-J18. Sepsis was defined as a diagnosis of either A40 (Streptococcal sepsis) or other sepsis (A41), with diagnoses recorded in primary care, hospital or death registries); Other Lower Respiratory Infections were defined as acute bronchitis, acute bronchiolitis, or unspecified acute lower respiratory infection, with respective ICD-10 diagnosis codes J20, J21 or J22. For analyses on severe pneumonia, individuals with pneumonia diagnoses recorded in primary care settings and as self-reports were excluded from the analyses; in other words, severe pneumonia was defined as having a diagnosis J12-J18 in hospital or death registries. There were approximately 110 000 individuals for available for the analyses of biomarkers with these diseases. The registry-based follow-up was from blood sampling in 2007-2010 through to 2016-2017, depending on UK Biobank assessment centre (approximately 872 000 person-years). For analyses on severe pneumonia, further analyses were conducted to demonstrate that the results were consistent for men and women, across different ages, and for people with and without chronic disease conditions at the time of the blood sampling. Specifically, the analyses on biomarker scores vs severe pneumonia were repeated when individuals with chronic respiratory and cardiometabolic diseases (cardiovascular disease, diabetes, lung cancer, chronic obstructive pulmonary disease, liver diseases, and renal failure) were omitted from the analyses. Analyses of biomarker scores were also conducted vs short-term risk of severe pneumonia, defined as pneumonia diagnoses recorded from hospital or death registries during the first 2 years after the blood samples were taken.

For biomarker association testing, Cox proportional-hazard regression models adjusted for age, sex, and assessment centre were used. The biomarkers were studied for the magnitude of association without and with scaling relative to albumin concentration. Results were plotted in magnitudes per standard deviation of each biomarker measure to allow direct comparison of association magnitudes. The relations of 3 specific plurality-biomarker-scores (scores calculated on the basis of a plurality of biomarkers) vs risk for severe pneumonia were examined in further detail, in form of analyses of different subsets of the study population (with and without individuals who had chronic respiratory or cardiometabolic disease at time of blood sampling, different age groups of the study population, men and women separately, and short-term compared to long-term risk of severe pneumonia). In addition, the same 3 plurality-biomarker-scores were plotted in form of gradient percentile plots, showing the proportion of individuals who developed severe pneumonia during follow-up when binning individuals into the percentiles of the biomarker levels. We also examined these 3 plurality-biomarker-scores using Kaplan-Meier plots of the cumulative risk for pneumonia during follow-up, for quintiles of the plurality-biomarker-scores and further stratified for extreme quantiles of the two biomarkers.

### Summary of results

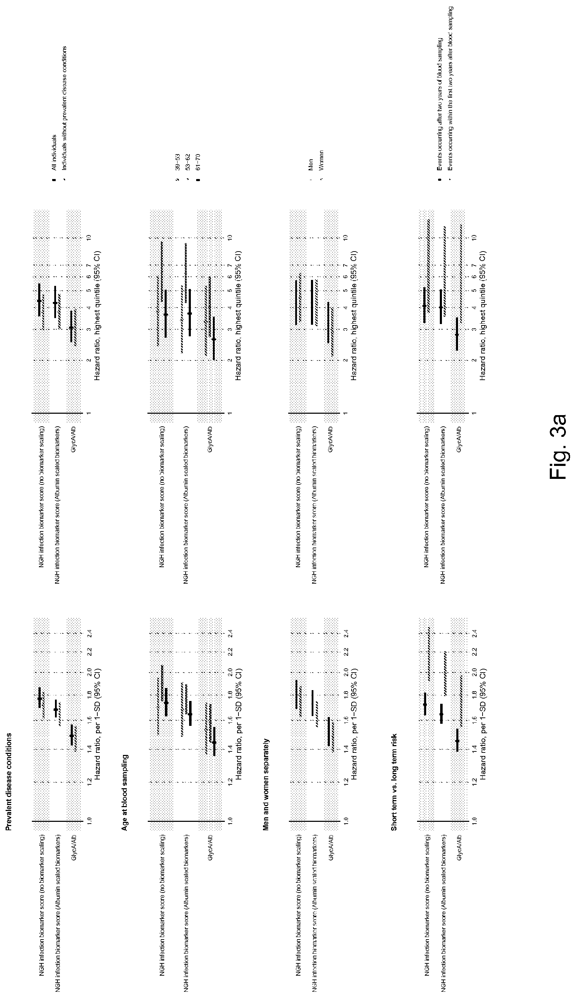

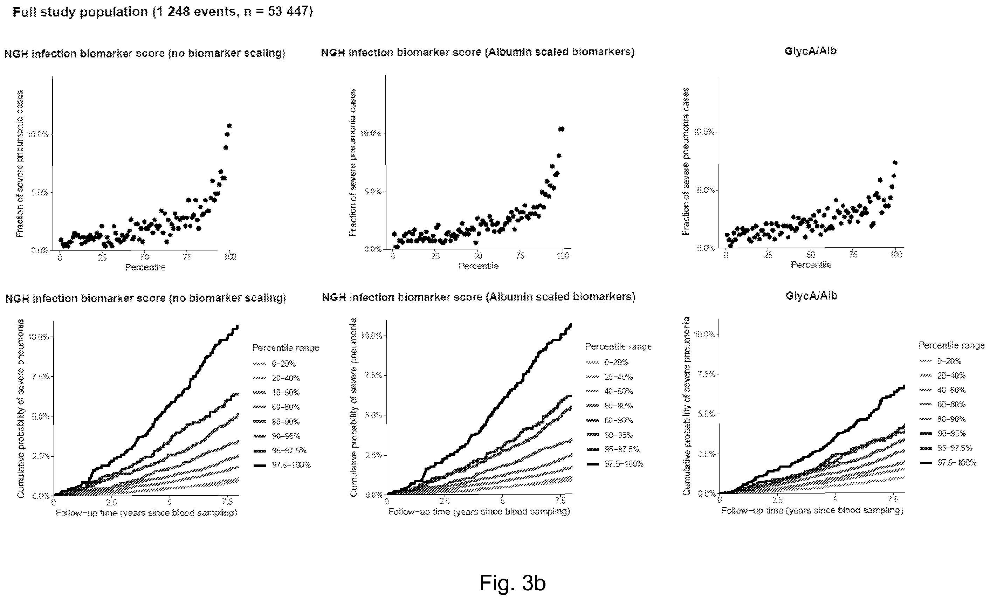

Baseline characteristics of the study population for biomarker analyses vs future disease risk are shown in Table 1. The number of disease cases for severe pneumonia is exemplified. Among the 108 449 study participants with complete data available to calculate the biomarker scores, there were 2531 pneumonia events recorded in hospital or death registries after the baseline blood sampling (median follow-up time 8.1 years). For other diseases studied here, the number of individuals who developed the given disease after the blood samples were taken are listed in the Figures that illustrate the results, and the clinical characteristics are similar to the data in Table 1. For the plurality-biomarker scores shown in Figure 3a and Figure 3b, only half of the study population was used in the plots, since the other half was used to derive the weights for the combinations of biomarkers in the plurality-biomarker scores.

**Table 1: Clinical characteristics of study participants.**

| | Severe Pneumonia (hospitalisation or death) | |
|---|---|---|
| | Incident cases | Controls |
| Individuals with NMR biomarker measures | 2531 | 105 918 |
| Age (median) | 62.0 | 57.0 |
| Females (%) | 44 % | 54 % |
| Cardiovascular disease (%) | 18 % | 6 % |
| Diabetes (%) | 10 % | 4 % |
| Lung cancer (%) | 1 % | 0.2 % |
| Chronic obstructive pulmonary disease (%) | 6 % | 1 % |
| Liver diseases (%) | 2 % | 1 % |
| Renal failure (%) | 4 % | 1 % |

### Biomarker associations for future diabetes and pneumonia when scaling concentrations relative to albumin concentration

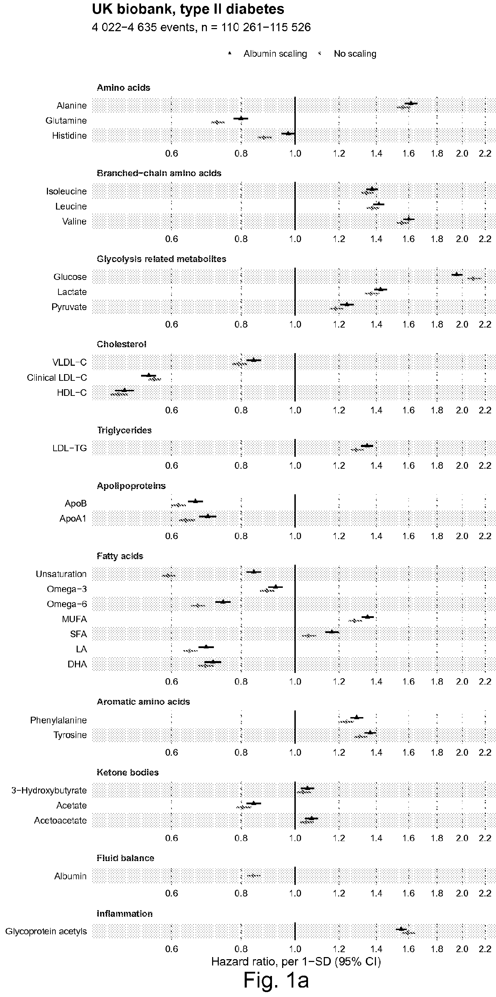

**Fig. 1a** shows the hazard ratios for 29 blood biomarkers for future onset of type 2 diabetes. The results are based on statistical analyses of over 110,000 individuals from the UK Biobank, out of whom over 4,000 developed type 2 diabetes during a median of 8.1 year follow-up. The results are shown for absolute concentration of the biomarkers (grey circles), and when the biomarker concentrations are scaled relative to the concentration of albumin measured from the same blood sample (black triangles). The analyses were adjusted for age, sex, and UK Biobank assessment centre in Cox proportional-hazard regression models. The results for both unscaled and albumin-scaled biomarkers are shown per 1-standard deviation increase in the given biomarker measure.

All the 29 biomarkers were statistically significantly associated with future onset of type 2 diabetes, both when analysed as absolute concentrations, and when the concentrations were scaled relative to albumin. All associations were in the same direction in relation to type 2 diabetes risk with and without scaling concentrations relative to albumin.

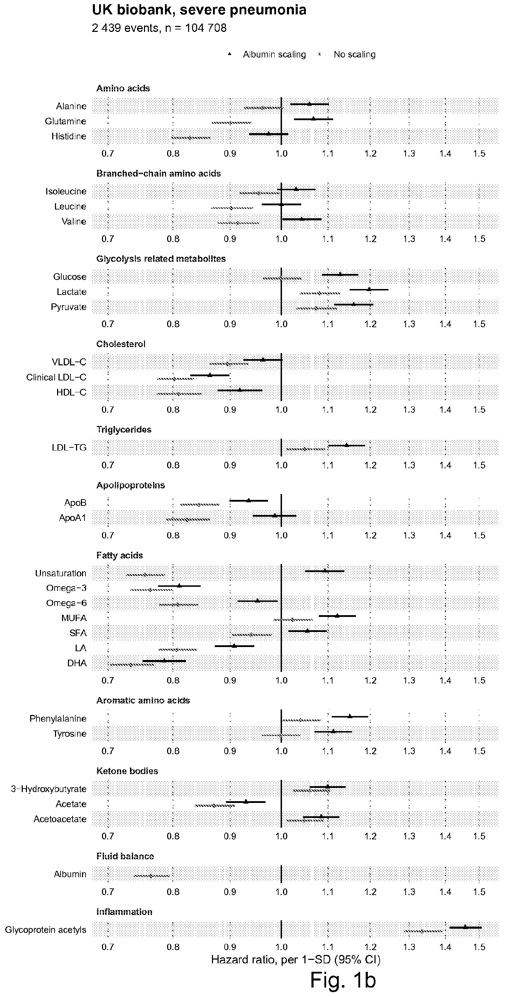

**Figure 1b** shows the hazard ratios for 29 blood biomarkers for future onset of severe pneumonia. The results are based on statistical analyses of over 100,000 individuals from the UK Biobank, out of whom over 2,400 developed severe pneumonia (defined as diagnosis J12-J18 in the national hospital registries or death records) during a median of 8.1 year follow-up. The results are shown for absolute concentration of the biomarkers (grey circles), and when the biomarker concentrations are scaled relative to the concentration of albumin measured from the same blood sample (black triangles). The analyses were adjusted for age, sex, and UK Biobank assessment centre in Cox proportional-hazard regression models. The results for both unscaled and albumin-scaled biomarkers are shown per 1-standard deviation increase in the given biomarker measure.

All 29 biomarkers were associated with future onset of severe pneumonia when analysed either as absolute concentrations, or when the concentrations were scaled relative to albumin. 23 out of the 29 biomarkers were statistically significantly associated with future onset of severe pneumonia when analysed as absolute concentrations. There were also 23 out biomarkers that were statistically significantly associated with future onset of severe pneumo- nia when the concentrations were scaled relative to albumin.

Overall, the analyses illustrate that a broad span of blood biomarkers measured by NMR spectroscopy are indicative of the risk for future type 2 diabetes and severe pneumonia in general population settings. The biomarker associations for type 2 diabetes are similar if the concentrations are scaled relative to albumin concentration. The biomarker associations for severe pneumonia are altered to some extend when concentrations are scaled relative to albumin, but many biomarkers are still strongly associated with risk for severe pneumonia after the albumin-scaling, or become even stronger associated with severe pneumonia after the albumin scaling.

### Abbreviations used in the Figures:

VLDL: Very-Low-Density Lipoprotein
LDL: Low-Density Lipoprotein
HDL: High-Density Lipoprotein
C: cholesterol
TG: Triglycerides
LA: Linoleic acid
DHA: Docosahexaenoic acid
SFA: Saturated fatty acids
MUFA: Monounsaturated fatty acids
SD: standard deviation

### EXAMPLE 2

The study population and statistical methods for Example 2 are as for Example 1.

### Summary of Results on Biomarkers for Infectious diseases and their complications

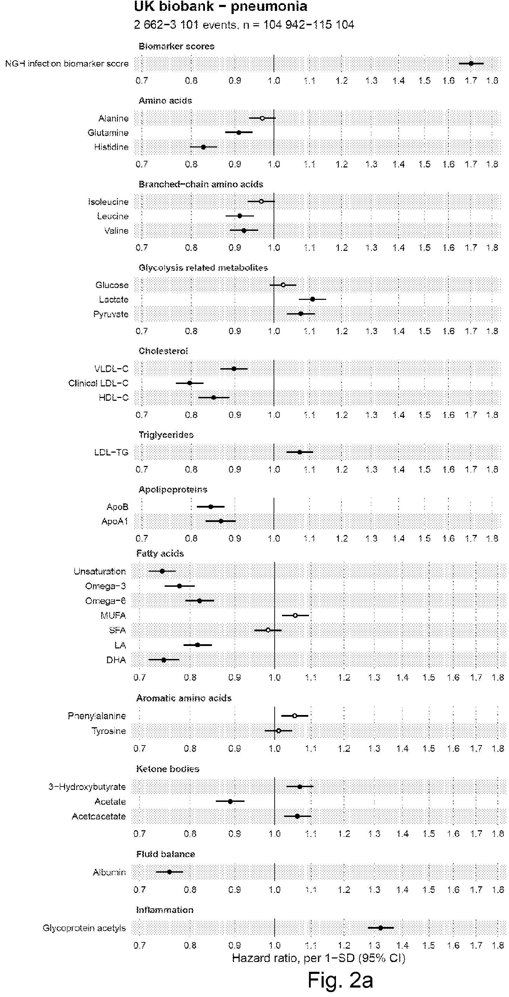

**Figure 2a** shows the hazard ratios for 29 blood biomarkers for future onset of pneumonia (either severe or non-severe), when the biomarkers are analysed in absolute concentrations. The association for a plurality-biomarker-score termed "NGH infection biomarker score", comprising a weighted sum of the individual biomarkers, is also shown. The results are based on statistical analyses of over 100,000 individuals from the UK Biobank, out of whom over 2,650 developed pneumonia (defined as diagnosis J12-J18 in the primary care records, or in the national hospital registries, or in the death records, or self-reported) during a median of 8.1 year follow-up. Filled circles denote that the P-value for association was P<0.001 (corresponding to multiple testing correction), and open circles that the P-value for association was P>0.001. The analyses were adjusted for age, sex, and UK Biobank assessment centre in Cox proportional-hazard regression models. The results are shown per 1-standard deviation increase in the given biomarker measure. 22 out of the 29 biomarkers were statistically significantly associated with future onset of pneumonia, based on P<0.001. The strongest association with risk for future development of pneumonia was observed for the plurality-biomarker-score termed "NGH infection biomarker score".

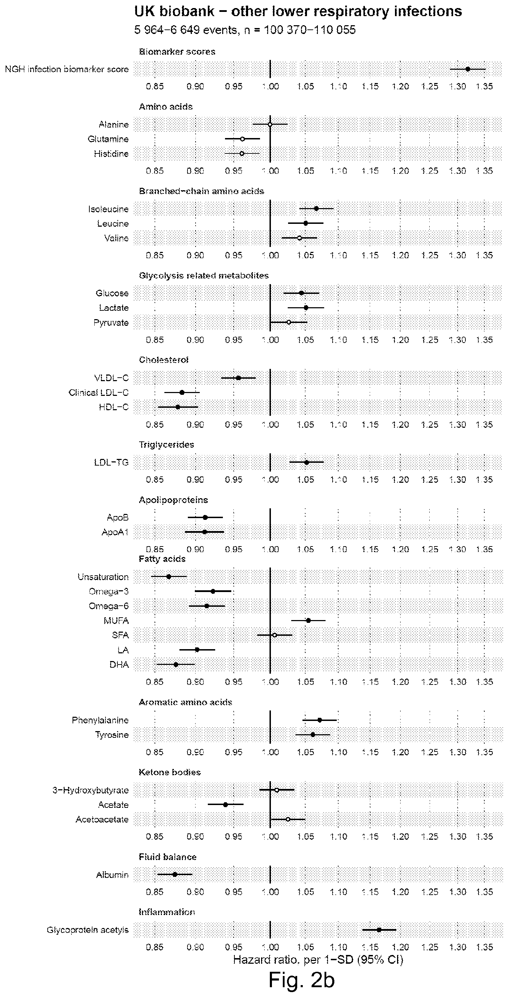

**Figure 2b** shows the relation of baseline biomarker levels to future development of Other Lower Respiratory Diseases (acute bronchitis, acute bronchiolitis, and Unspecified acute lower respiratory infection), when the biomarker concentrations are analysed in absolute concentrations. shows the hazard ratios for 29 blood biomarkers for future onset of sepsis, when the biomarkers are analysed in absolute concentrations. The association for a plurality-biomarker-score termed "NGH infection biomarker score", comprising a weighted sum of the individual biomarkers, is also shown. The results are based on statistical analyses of over 100,000 individuals from the UK Biobank, out of whom over 5,900 developed sepsis (defined as diagnosis J20 (acute bronchitis) or J21 (acute bronchiolitis) or J22(Other acute lower respiratory infections) in the primary care records, self-reported, or in the national hospital registries, or in the death records) during a median of 8.1 year follow-up. Filled circles denote that the P-value for association was P<0.001, and open circles that the P-value for association was P>0.001. The analyses were adjusted for age, sex, and UK Biobank assessment centre in Cox proportional-hazard regression models. The results are shown per 1-standard deviation increase in the given biomarker measure. 21 out of the 29 biomarkers were statistically significantly associated with future onset of sepsis, based on P<0.001. The strongest associa- tion with risk for future development of sepsis was observed for the plurality-biomarker-score termed "NGH infection biomarker score".

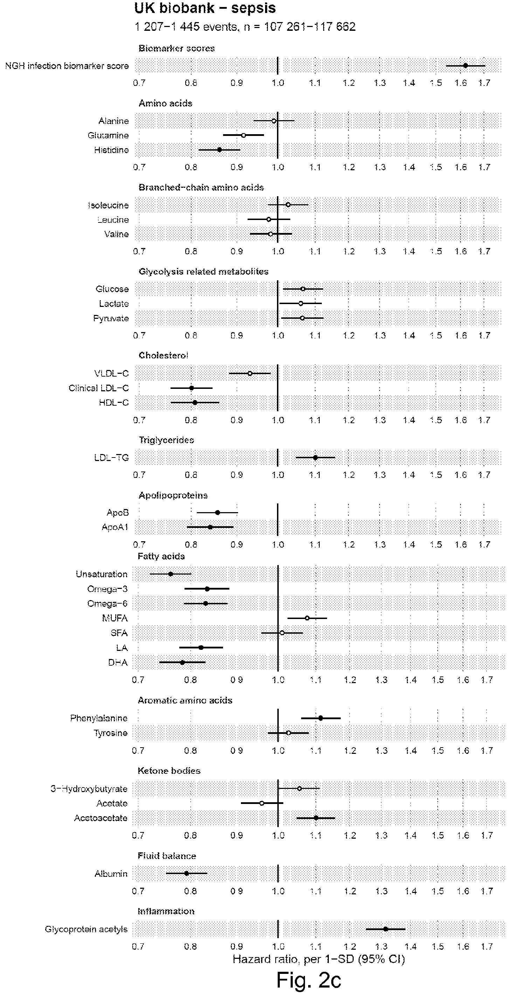

**Figure 2c** shows the hazard ratios for 29 blood biomarkers for future onset of sepsis, when the biomarkers are analysed in absolute concentrations. The association for a plurality-biomarker-score termed "NGH infection biomarker score", comprising a weighted sum of the individual biomarkers, is also shown. The results are based on statistical analyses of over 100,000 individuals from the UK Biobank, out of whom over 1,250 developed sepsis (defined as diagnosis A40 (Streptococcal sepsis) or A41 (Other sepsis) in the primary care records, self-reported, or in the national hospital registries, or in the death records) during a median of 8.1 year follow-up. Filled circles denote that the P-value for association was P<0.001, and open circles that the P-value for association was P>0.001. The analyses were adjusted for age, sex, and UK Biobank assessment centre in Cox proportional-hazard regression models. The results are shown per 1-standard deviation increase in the given biomarker measure. 15 out of the 29 biomarkers were statistically significantly associated with future onset of sepsis, based P<0.001. The strongest association with risk for future development of sepsis was observed for the plurality-biomarker-score termed "NGH infection biomarker score".

**Figure 3a** shows the relation of baseline biomarker levels for 3 plurality-biomarker-scores to future development of severe pneumonia. Specifically, the plurality biomarker the following: 1) a weighted combination of up to 29 biomarkers in absolute concentrations (denoted NGH infection biomarker score; no biomarker scaling), 2) a weighted combination of up to 29 biomarkers in concentrations scaled relative to albumin (denoted NGH infection biomarker score; albumin scaled biomarkers), and 3) Glycoprotein acetyls scaled to albumin concentration (denoted GlycA/Alb). The weighted combinations of biomarkers used in the plurality-biomarker-scores were derived using statistical regression analyses and machine learning, based on 50% of the study population (derivation set; approximately 53,000 individuals). The predictive performance of the 3 plurality-biomarker-scores was then evaluated in the other 50% of the study population (validation set; 53477 individuals) to avoid overfitting of the results. The associations of the 3 plurality-biomarker-scores are shown for all individuals in the validation part of the study population, and in the subset of individuals without chronic respiratory or cardiometabolic disease at baseline. The results are also shown in different age groups (divided in age-tertiles of the study participants when they gave the blood samples), and separately for men and women. The relation of the baseline biomarker levels for 3 different plurality-biomarker-scores with severe pneumonia occurring within 2 years after the blood samples were taken is also shown.

These results show the following:
- that the 3 plurality-biomarker-scores are stronger associated with future development than any single biomarker (see Figure 1b for results on single biomarkers).
- that Glycoproteins relative to albumin, as well as a plurality-biomarker-score based on biomarkers scaled relative to albumin concentration, are strongly predictive of future development of severe pneumonia
- that the results are similar irrespective of age of study participants.
- that the results are similar for men and women.
- that the results are even stronger for prediction of short-term risk for severe pneumonia (disease occurring first 2 years after blood sampling), compared to long-term risk for severe pneumonia (disease occurring on average up to 8 years after the blood sampling)

**Figure 3b** shows the risk gradient for future severe pneumonia according to percentiles of the 3 plurality-biomarker-scores (upper half of the figure). Each dot corresponds to approximately 500 individuals. are also shown. We also calculated the cumulative risk for severe pneumonia after the blood samples were taken in various quantiles of the 3 plurality-biomarker-scores using Kaplan-Meier plots (lower half of the figure). The plots are shown for the validation set part of the study population, i.e. 50% which was not included for derivation of the plurality-biomarker-scores (n=53477 individuals).

These results show the following:
- The relation of the 3 plurality-biomarker-scores with risk for future onset of severe pneumonia is particularly elevated for individuals with the very highest levels of the plurality-biomarker-scores, i.e. non-linear effects.
- The increase in risk for individuals with the very highest levels of the plurality-biomarker-scores is present within the first few years after the time of blood sampling.

It is obvious to a person skilled in the art that with the advancement of technology, the basic idea may be implemented in various ways.

The embodiments described hereinbefore may be used in any combination with each other. Several of the embodiments may be combined together to form a further embodiment. A method, a product, or a use, disclosed herein, may comprise at least one of the embodiments described hereinbefore. It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item refers to one or more of those items. The term "comprising" is used in this specification to mean including the feature(s) or act(s) followed thereafter, without excluding the presence of one or more additional features or acts.

## Claims

1. A method for determining whether a subject has a disease or condition or is at risk of developing a disease or condition, wherein the method comprises
determining in a biological sample obtained from the subject a quantitative value of at least one biomarker relative to a quantitative value of albumin in the biological sample; and
comparing the quantitative value(s) of the at least one biomarker to a control sample or to a control value;
wherein an increase or a decrease in the quantitative value(s) of the at least one biomarker, when compared to the control sample or to the control value, is/are indicative of the subject having the disease or condition or having an increased risk of developing the disease or condition;
wherein the biological sample is a dry blood sample, a dry sample obtainable from blood, or obtainable from a dry blood sample;
wherein the quantitative value of the at least one biomarker and the quantitative value of the albumin are measured using nuclear magnetic spectroscopy; and
wherein the at least one biomarker comprises or is glycoprotein acetyls.

2. The method according to claim 1, wherein the quantitative value of the at least one biomarker relative to the quantitative value of the albumin in the biological sample is determined by calculating a ratio of an initial quantitative value of the at least one biomarker and the quantitative value of the albumin in the biological sample.

3. The method according to claim 1 or 2, wherein the dry blood sample is a dried blood spot.

4. The method according to any one of claims 1 - 3, wherein the dry blood sample or the dry sample obtainable from blood is obtainable from a fingertip of the subject, sampling of capillary blood from the upper arm of the subject, and/or by venepuncture of the subject.

5. The method according to any one of claims 1 - 4, wherein the biological sample is obtainable from the dry blood sample or from the dry sample obtainable from blood by extracting at least a part of the blood or one or more components thereof from the dry blood sample or from the dry sample obtainable from blood into a solvent.

6. The method according to any one of claims 1 - 5, wherein the method comprises determining in the biological sample quantitative values of a plurality of biomarkers, such as two, three, four, five or more biomarkers.

7. The method according to any one of claims 1 - **6,** wherein the method comprises determining in a biological sample obtained from the subject a quantitative value of at least one biomarker of the following relative to the quantitative value of the albumin in the biological sample:
- glycoprotein acetyls,
- omega-6 fatty acids,
- monounsaturated fatty acids,
- saturated fatty acids,
- omega-3 fatty acids,
- apolipoprotein A1 (ApoA1),
- docosahexaenoic acid (DHA),
- leucine,
- acetate,
- alanine,
- apolipoprotein B (ApoB),
- glutamine,
- isoleucine,
- linoleic acid,
- phenylalanine,
- tyrosine,
- degree of unsaturation of fatty acids,
- valine,
- histidine,
- cholesterol in HDL (HDL-C),
- glucose,
- acetoacetate,
- 3-hydroxybutyrate,
- cholesterol in LDL (LDL-C),
- lactate,
- triglycerides in LDL (LDL-TG),
- pyruvate, or
- cholesterol in VLDL (VLDL-C).

8. The method according to any one of claims 1 - 7, wherein the method comprises determining in the biological sample obtained from the subject a quantitative value of at least one biomarker of the following relative to the quantitative value of the albumin in the biological sample:
- omega-6 fatty acids,
- monounsaturated fatty acids,
- saturated fatty acids, or
- omega-3 fatty acids; and/or
a quantitative value of at least one biomarker of the following relative to the quantitative value of the albumin in the biological sample:
- apolipoprotein A1 (ApoA1),
- docosahexaenoic acid (DHA),
- leucine,
- acetate,
- alanine,
- apolipoprotein B (ApoB),
- glutamine,
- isoleucine,
- linoleic acid,
- phenylalanine,
- tyrosine,
- degree of unsaturation of fatty acids,
- valine,
- histidine; and/or
a quantitative value of at least one biomarker of the following relative to the quantitative value of the albumin in the biological sample:
- cholesterol in HDL (HDL-C),
- glucose,
- acetoacetate,
- 3-hydroxybutyrate,
- cholesterol in LDL (LDL-C),
- lactate,
- triglycerides in LDL (LDL-TG),
- pyruvate, or
- cholesterol in VLDL (VLDL-C).

9. The method according to any one of claims 1 **-** 8, wherein the disease is an infectious disease or a complication thereof, such as sepsis, pneumonia, optionally severe pneumonia, or other lower respiratory infection; or diabetes.

10. The method according to any one of claims 1 - 9, wherein the dry blood sample is a dried whole blood sample, a dried plasma sample, a dried serum sample, or a dried sample derived therefrom.

11. The method according to any one of claims 1 - 10, wherein the method further comprises determining whether the subject has the disease or condition or is at risk of developing the disease or condition using a risk score, hazard ratio, and/or predicted absolute risk calculated on the basis of the quantitative value(s) of the at least one biomarker or of the plurality of the biomarkers relative to the quantitative value of the albumin.

## Patentansprüche

1. Verfahren zum Bestimmen, ob ein Subjekt an einer Krankheit oder einem Zustand leidet oder ein Risiko hat, eine Krankheit oder einen Zustand zu entwickeln, wobei das Verfahren umfasst
bestimmen, in einer von dem Subjekt erhaltenen biologischen Probe, eines quantitativen Werts von mindestens einem Biomarker in Relation zu dem quantitativen Wert des Albumins in der biologischen Probe; und
vergleichen des oder der quantitativen Werte des mindestens einen Biomarkers mit einer Kontrollprobe oder mit einem Kontrollwert;
wobei eine Erhöhung oder eine Verringerung des oder der quantitativen Werte des mindestens einen Biomarkers im Vergleich zu der Kontrollprobe oder zu dem Kontrollwert ein Hinweis darauf ist, dass das Subjekt die Krankheit oder den Zustand aufweist oder ein erhöhtes Risiko hat, die Krankheit oder den Zustand zu entwickeln;
wobei die biologische Probe eine Trockenblutprobe ist, eine aus Blut gewinnbare Trockenprobe ist oder aus einer Trockenblutprobe gewinnbar ist;
wobei der quantitative Wert des mindestens einen Biomarkers und der quantitative Wert des Albumins mittels nuklearmagnetischer Spektroskopie gemessen werden; und
wobei der mindestens eine Biomarker Glykoproteinacetyle umfasst oder Glykoproteinacetyle ist.

2. Verfahren nach Anspruch 1, wobei der quantitative Wert des mindestens einen Biomarkers in Relation zu dem quantitativen Wert des Albumins in der biologischen Probe dadurch bestimmt wird, dass ein Verhältnis des anfänglichen quantitativen Wertes des mindestens einen Biomarkers zu dem quantitativen Wert des Albumins in der biologischen Probe berechnet wird.

3. Verfahren nach Anspruch 1 oder 2, wobei die Trockenblutprobe ein getrockneter Blutfleck ist.

4. Verfahren nach einem der Ansprüche 1 bis 3, wobei die Trockenblutprobe oder die aus Blut gewinnbare Trockenprobe aus einer Fingerkuppe des Subjekts, durch Probenahme von Kapillarblut aus dem Oberarm des Subjekts und oder durch Venenpunktion bei dem Subjekt gewinnbar ist.

5. Verfahren nach einem der Ansprüche 1 bis 4, wobei die biologische Probe aus der Trockenblutprobe oder aus der aus Blut gewinnbaren Trockenprobe dadurch gewinnbar ist, indem zumindest ein Teil des Blutes oder eine oder mehrere Komponenten davon aus der Trockenblutprobe oder aus der aus Blut gewinnbaren Trockenprobe in ein Lösungsmittel extrahiert wird.

6. Verfahren nach einem der Ansprüche 1 bis 5, wobei das Verfahren umfasst, in der biologischen Probe quantitative Werte einer Mehrzahl von Biomarkern, wie zwei, drei, vier, fünf oder mehr Biomarker, zu bestimmen.

7. Verfahren nach einem der Ansprüche 1 bis 6, wobei das Verfahren umfasst, in einer von dem Subjekt erhaltenen biologischen Probe einen quantitativen Wert von mindestens einem der folgenden Biomarker in Relation zu dem quantitativen Wert des Albumins in der biologischen Probe zu bestimmen:
- Glykoproteinacetyle,
- Omega-6-Fettsäuren,
- einfach ungesättigte Fettsäuren,
- gesättigte Fettsäuren,
- Omega-3-Fettsäuren,
- Apolipoprotein Al (ApoAl),
- Docosahexaensäure (DHA),
- Leucin,
- Acetat,
- Alanin,
- Apolipoprotein B (ApoB),
- Glutamin,
- Isoleucin,
- Linolsäure,
- Phenylalanin,
- Tyrosin,
- Grad der Ungesättigtheit von Fettsäuren,
- Valin,
- Histidin,
- Cholesterin in HDL (HDL-C),
- Glukose,
- Acetoacetat,
- 3-Hydroxybutyrat,
- Cholesterin in LDL (LDL-C),
- Laktat,
- Triglyceride in LDL (LDL-TG),
- Pyruvat, oder
- Cholesterin in VLDL (VLDL-C).

8. Verfahren nach einem der Ansprüche 1 bis 7, wobei das Verfahren umfasst, in der von dem Subjekt erhaltenen biologischen Probe einen quantitativen Wert von mindestens einem der folgenden Biomarker in Relation zu dem quantitativen Wert des Albumins in der biologischen Probe zu bestimmen:
- Omega-6-Fettsäuren,
- einfach ungesättigte Fettsäuren,
- gesättigte Fettsäuren, oder
- Omega-3-Fettsäuren; und oder
einen quantitativen Wert von mindestens einem der folgenden Biomarker in Relation zu dem quantitativen Wert des Albumins in der biologischen Probe:
- Apolipoprotein Al (ApoAl),
- Docosahexaensäure (DHA),
- Leucin,
- Acetat,
- Alanin,
- Apolipoprotein B (ApoB),
- Glutamin,
- Isoleucin,
- Linolsäure,
- Phenylalanin,
- Tyrosin,
- Grad der Ungesättigtheit von Fettsäuren,
- Valin,
- Histidin; und oder
einen quantitativen Wert von mindestens einem der folgenden Biomarker in Relation zu dem quantitativen Wert des Albumins in der biologischen Probe:
- Cholesterin in HDL (HDL-C),
- Glukose,
- Acetoacetat,
- 3-Hydroxybutyrat,
- Cholesterin in LDL (LDL-C),
- Laktat,
- Triglyceride in LDL (LDL-TG),
- Pyruvat, oder
- Cholesterin in VLDL (VLDL-C).

9. Verfahren nach einem der Ansprüche 1 bis 8, wobei die Krankheit eine Infektionskrankheit oder eine Komplikation davon ist, wie Sepsis, Pneumonie, gegebenenfalls schwere Pneumonie oder andere untere Atemwegsinfektion, oder Diabetes.

10. Verfahren nach einem der Ansprüche 1 bis 9, wobei die Trockenblutprobe eine getrocknete Vollblutprobe, eine getrocknete Plasmaprobe, eine getrocknete Serumprobe oder eine getrocknete davon abgeleitete Probe ist.

11. Verfahren nach einem der Ansprüche 1 bis 10, wobei das Verfahren ferner umfasst, zu bestimmen, ob das Subjekt die Krankheit oder den Zustand aufweist oder ein Risiko hat, die Krankheit oder den Zustand zu entwickeln, unter Verwendung eines Risikoscores, einer Hazard Ratio und oder eines vorhergesagten absoluten Risikos, jeweils berechnet auf der Grundlage des oder der quantitativen Werte des mindestens einen Biomarkers oder der Mehrzahl der Biomarker relativ zu dem quantitativen Wert des Albumins.

## Revendications

1. Procédé pour déterminer si un sujet présente une maladie ou une pathologie ou présente un risque de développer une maladie ou une pathologie, dans lequel le procédé comprend
la détermination, dans un échantillon biologique obtenu chez le sujet, d'une valeur quantitative d'au moins un biomarqueur par rapport à une valeur quantitative d'albumine dans l'échantillon biologique ; et
la comparaison de la ou des valeurs quantitatives de l'au moins un biomarqueur à un échantillon témoin ou à une valeur témoin ;
dans lequel une augmentation ou une diminution de la ou des valeurs quantitatives de l'au moins un biomarqueur, par rapport à l'échantillon témoin ou à la valeur témoin, est/sont indicative(s) que le sujet présente la maladie ou la pathologie ou présente un risque accru de développer la maladie ou la pathologie ;
dans lequel l'échantillon biologique est un échantillon de sang sec, un échantillon sec pouvant être obtenu à partir de sang, ou pouvant être obtenu à partir d'un échantillon de sang sec ;
dans lequel la valeur quantitative de l'au moins un biomarqueur et la valeur quantitative de l'albumine sont mesurées en utilisant une spectroscopie magnétique nucléaire ; et
dans lequel l'au moins un biomarqueur comprend ou est des acétyles de glycoprotéine.

2. Procédé selon la revendication 1, dans lequel la valeur quantitative de l'au moins un biomarqueur par rapport à la valeur quantitative de l'albumine dans l'échantillon biologique est déterminée par le calcul d'un rapport d'une valeur quantitative initiale de l'au moins un biomarqueur et de la valeur quantitative de l'albumine dans l'échantillon biologique.

3. Procédé selon la revendication 1 ou 2, dans lequel l'échantillon de sang sec est un caillot de sang séché.

4. Procédé selon l'une quelconque des revendications 1 à 3, dans lequel l'échantillon de sang sec ou l'échantillon sec pouvant être obtenu à partir de sang peut être obtenu en provenance d'un bout de doigt du sujet, d'un échantillonnage de sang capillaire provenant du bras du sujet, et/ou par une ponction veineuse du sujet.

5. Procédé selon l'une quelconque des revendications 1 à 4, dans lequel l'échantillon biologique peut être obtenu à partir de l'échantillon de sang sec ou à partir de l'échantillon sec pouvant être obtenu à partir de sang par l'extraction d'au moins une partie du sang ou d'un ou plusieurs composants de celui-ci de l'échantillon de sang sec ou de l'échantillon sec pouvant être obtenu à partir de sang dans un solvant.

6. Procédé selon l'une quelconque des revendications 1 à 5, dans lequel le procédé comprend la détermination, dans l'échantillon biologique, de valeurs quantitatives d'une pluralité de biomarqueurs, par exemple deux, trois, quatre, cinq biomarqueurs ou plus.

7. Procédé selon l'une quelconque des revendications 1 à 6, dans lequel le procédé comprend la détermination, dans un échantillon biologique obtenu chez le sujet, d'une valeur quantitative d'au moins un biomarqueur des éléments suivants par rapport à la valeur quantitative de l'albumine dans l'échantillon biologique :
- acétyles de glycoprotéine,
- acides gras oméga-6,
- acides gras mono-insaturés,
- acides gras saturés,
- acides gras oméga-3,
- apolipoprotéine A1 (ApoA1),
- acide docosahexaénoïque (DHA),
- leucine,
- acétate de carbone,
- alanine,
- apolipoprotéine B (ApoB),
- glutamine,
- isoleucine,
- acide linoléique,
- phénylalanine,
- tyrosine,
- degré d'insaturation des acides gras,
- valine,
- histidine,
- cholestérol HDL (HDL-C),
- glucose,
- acétoacétate,
- 3-hydroxybutyrate,
- cholestérol LDL (LDL-C),
- lactate,
- triglycérides LDL (LDL-TG),
- pyruvate, ou
- cholestérol VLDL (VLDL-C).

8. Procédé selon l'une quelconque des revendications 1 à 7, dans lequel le procédé comprend la détermination, dans l'échantillon biologique obtenu chez le sujet, d'une valeur quantitative d'au moins un biomarqueur des éléments suivants par rapport à la valeur quantitative de l'albumine dans l'échantillon biologique :
- acides gras oméga-6,
- acides gras mono-insaturés,
- acides gras saturés, ou
- acides gras oméga -3 ; et/ou
une valeur quantitative d'au moins un biomarqueur des éléments suivants par rapport à la valeur quantitative de l'albumine dans l'échantillon biologique :
- apolipoprotéine A1 (ApoA1) - acide docosahexaénoïque (DHA),
- leucine,
- acétate de carbone,
- alanine,
- apolipoprotéine B (ApoB),
- glutamine,
- isoleucine,
- acide linoléique,
- phénylalanine,
- tyrosine,
- degré d'insaturation des acides gras,
- valine,
- histidine ; et/ou
une valeur quantitative d'au moins un biomarqueur des éléments suivants par rapport à la valeur quantitative de l'albumine dans l'échantillon biologique :
- cholestérol HDL (HDL-C),
- glucose,
- acétoacétate,
- 3-hydroxybutyrate,
- cholestérol LDL (LDL-C),
- lactate,
- triglycérides LDL (LDL-TG),
- pyruvate, ou
- cholestérol VLDL (VLDL-C).

9. Procédé selon l'une quelconque des revendications 1 à 8, dans lequel la maladie est une maladie infectieuse ou une complication de celle-ci, telle qu'une sepsie, une pneumonie, éventuellement une pneumonie grave, ou une autre infection des voies respiratoires inférieures ; ou un diabète.

10. Procédé selon l'une quelconque des revendications 1 à 9, dans lequel l'échantillon de sang sec est un échantillon de sang total séché, un échantillon de plasma séché, un échantillon de sérum séché, ou un échantillon séché dérivé de ceux-ci.

11. Procédé selon l'une quelconque des revendications 1 à 10, dans lequel le procédé comprend en outre le fait de déterminer si le sujet présente la maladie ou la pathologie ou présente un risque de développer la maladie ou la pathologie en utilisant un score de risque, un rapport de danger et/ou un risque absolu prédit calculé sur la base de la ou des valeurs quantitatives de l'au moins un biomarqueur ou de la pluralité des biomarqueurs par rapport à la valeur quantitative de l'albumine.
